# FASCICULE DE BREVET EUROPEEN

(11) **EP 0 889 758 B1**
(45) Date de publication et mention de la délivrance du brevet: **17.07.2002**
(21) Numéro de dépôt: 97919450.3
(22) Date de dépôt: 01.04.1997
(51) Int. Cl.: B05B 11/02

(54) **DISPOSITIF DE DISTRIBUTION D'UNE DOSE UNIQUE DE PRODUIT FLUIDE**
VERTEILUNGSEINRICHTUNG EINER EINMALIGEN DOSIS EINES FLÜSSIGEN PRODUKTES
DEVICE FOR DISPENSING A SINGLE DOSE OF FLUID

(30) Priorité: 02.04.1996 FR 9604119
(43) Date de publication de la demande: 13.01.1999
(73) Titulaire: VALOIS S.A., 27110 Le Neubourg (FR)
(72) Inventeur: MARTIN, Claude-Georges, F-27110 Yville (FR); PETIT, Ludovic, F-27110 Vitot (FR)
(74) Mandataire: CAPRI SARL
(86) Numéro de dépôt international: FR9700575
(87) Numéro de publication internationale: WO9736691

(56) Documents cités:
- WO-A-91/13281
- DE-A- 4 412 041
- FR-A- 2 714 624
- US-A- 5 005 732

## Description

La présente invention concerne un dispositif de distribution d'une dose prédéterminée d'un produit fluide. Plus particulièrement, l'invention concerne un dispositif contenant une dose unique de produit et jetable après émission de cette dose. Le produit à projeter peut être un médicament, un cosmétique, ou similaire. Le dispositif selon l'invention est plus spécialement destiné aux pulvérisations nasales, mais pourrait être utiliser dans d'autres buts, telle que le pulvérisation auriculaire, sublinguale, etc.

De tels dispositifs sont connus dans l'état de l'art. Par exemple, le document EP-A-0 311 863 décrit un dispositif comportant un réservoir cylindrique de produit fluide dans lequel coulisse un piston solidaire d'un poussoir d'actionnement. Ce dispositif comporte en outre un passage de sortie qui communique avec le réservoir de produit. Mais ce passage de sortie n'est pas étanche à l'air de sorte que le produit contenu dans le réservoir peut s'oxyder ou se polluer au contact de l'air pendant son stockage.

Le document WO 93/00172 prévoit, pour résoudre ce problème, de disposer un piston mobile dans le réservoir cylindrique de telle sorte qu'en position de stockage, il coupe la communication entre le passage de sortie et le réservoir, et dans une position d'actionnement il met en communication ledit passage de sortie avec ledit réservoir, ledit piston étant maintenu dans sa position de stockage par coincement et n'étant déplacé vers sa position d'actionnement, pour libérer le passage de sortie, que s'il règne dans le réservoir une pression suffisante pour le décoincer. Cette mise en oeuvre, qui fonctionne de manière satisfaisante, présente toutefois un certain nombre d'inconvénients. Ainsi, pour pouvoir déterminer à l'avance le niveau de pression suffisante pour décoincer le piston, les dimensions de ce piston doivent être très précises pour assurer un coincement efficace en position de stockage et une libération garantie lors de l'actionnement du dispositif. Ceci peut s'avérer relativement difficile en raison des tolérances de dimension apparaissant lors de la fabrication des diverses pièces constitutives du dispositif. En outre, la fabrication et le montage du piston lui-même sont relativement compliqués et donc coûteux, ce qui a pour effet de renchérir sensiblement le coût du dispositif, particulièrement lorsque celui-ci est jetable après un actionnement unique. De même, la présence de ce piston, qui est un élément mobile par rapport à toutes les autres pièces constitutives du dispositif, représente une complication et donc une augmentation du coût non souhaitable pour la fabrication et le montage du dispositif.

Le document WO 91/13281 décrit un dispositif comportant les caractéristiques exposées dans le préambule de la revendication 1.

La présente invention a pour but de réaliser un dispositif du type précité qui ne présente pas les inconvénients ci-dessus. En particulier la présente invention a pour but de fournir un tel dispositif qui soit simple et peu coûteux à fabriquer et à monter, ce qui est particulièrement avantageux pour des dispositifs jetables après un actionnement unique. La présente invention a également pour but de fournir un tel dispositif qui garantisse une étanchéité totale pendant le stockage et une distribution complète de la dose de produit lors de son actionnement. La présente invention a également pour but de fournir un tel dispositif dans lequel le volume mort est minimalisé. La présente invention a encore pour but de fournir un tel dispositif qui comporte un minimum de pièces mobile indépendamment les unes par rapport aux autres.

La présente invention a donc pour objet un dispositif de distribution d'une dose unique de produit fluide, comportant un réservoir contenant ladite dose de produit fluide, un piston coulissant de manière étanche dans ledit réservoir entre une position de stockage dans laquelle il isole ledit réservoir, et une position d'actionnement, un organe d'actionnement du piston, un passage de sortie pour relier ledit réservoir à un orifice de sortie, et un gicleur disposé dans le passage de sortie pour pulvériser la dose de produit, ledit piston comportant un passage traversant qui est obturé de manière étanche, dans la position de stockage du piston, par un organe d'obturation, ledit organe d'obturation étant adapté à ouvrir ledit passage traversant sous l'effet d'une pression d'un niveau prédéterminé créée à l'intérieur du réservoir, de telle manière à permettre l'expulsion de ladite dose de produit fluide à travers ledit passage traversant, caractérisé en ce que ledit organe d'obturation est solidaire dudit gicleur, ledit gicleur étant mobile entre une position initiale correspondant à la position d'obturation de l'organe d'obturation, et une position finale correspondant à la position d'ouverture de l'organe d'obturation.

De préférence, ledit organe d'obturation est mobile entre une position d'obturation, dans laquelle il est disposé dans ledit passage traversant, et une position d'ouverture, dans laquelle il est expulsé hors dudit passage traversant.

Avantageusement, ledit passage traversant est au moins partiellement cylindrique et ledit organe d'obturation a des dimensions extérieures supérieures à la section transversale dudit passage traversant , de sorte qu'il est maintenu en position d'obturation par coincement, ledit organe d'obturation étant expulsé hors dudit passage traversant sous l'effet de ladite pression suffisante créée dans le réservoir.

Selon un mode de réalisation particulier de l'invention, ledit passage traversant comporte une cavité adapté à recevoir ledit organe d'obturation dans sa position d'obturation.

De préférence, les parois formant le passage traversant du piston sont déformables sous l'effet d'une pression suffisante pour permettre l'expulsion dudit organe d'obturation.

En particulier, ledit organe d'obturation peut être une bille.

Avantageusement, le gicleur comporte une partie d'extrémité s'étendant dans ledit réservoir lorsque le gicleur est dans sa position initiale, ladite partie d'extrémité étant destinée à remplir au moins partiellement le volume mort créé dans le passage traversant lorsque le gicleur est dans sa position finale.

Avantageusement, ledit réservoir est en verre.

De préférence, ledit piston est fixe par rapport audit organe d'actionnement et se déplace dans le réservoir ensemble avec ledit organe d'actionnement .

Avantageusement, ledit passage traversant comporte une première partie sensiblement cylindrique et une seconde partie sensiblement cylindrique, la section transversale transversale de ladite seconde partie étant supérieure à celle de ladite première partie, ledit organe d'obturation ayant des dimensions extérieures qui sont supérieures à ladite section de la première partie et inférieures à ladite section transversale de la seconde partie.

D'autres caractéristiques et avantages de la présente invention apparaîtront au cours de la description suivante de l'invention donnée à titre d'exemple non limitatif, en regard des dessins joints, sur lesquels :
- la figure 1 est une vue schématique de face en coupe longitudinale d'une première forme de réalisation du dispositif qui n'est pas couverte par l'invention, en position de stockage,
- la figure 2 est une vue schématique de côté en coupe longitudinale du dispositif de la figure 1, en position de stockage,
- la figure 3 est une vue similaire à celle de la figure 1, en pcsition de fin d'actionnement du dispositif,
- la figure 4 est une vue similaire à celle de la figure 1, représentant une forme de réalisation du dispositif selon l'invention, en position de stockage, et
- la figure 5 est une vue similaire à celle de la figure 4, en position de fin d'actionnement du dispositif.

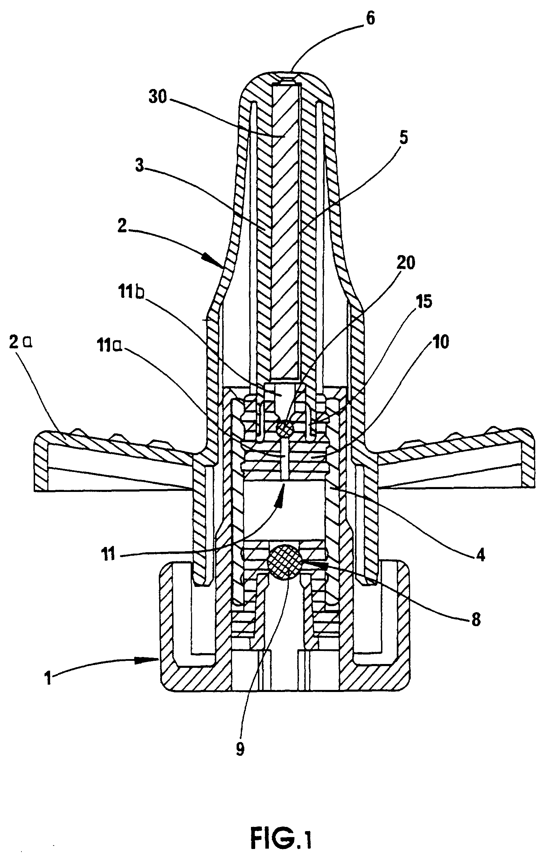

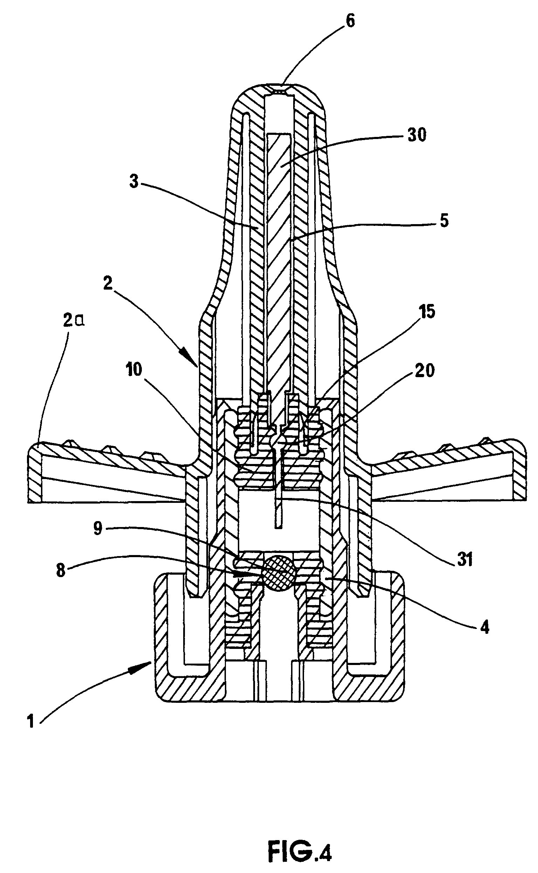

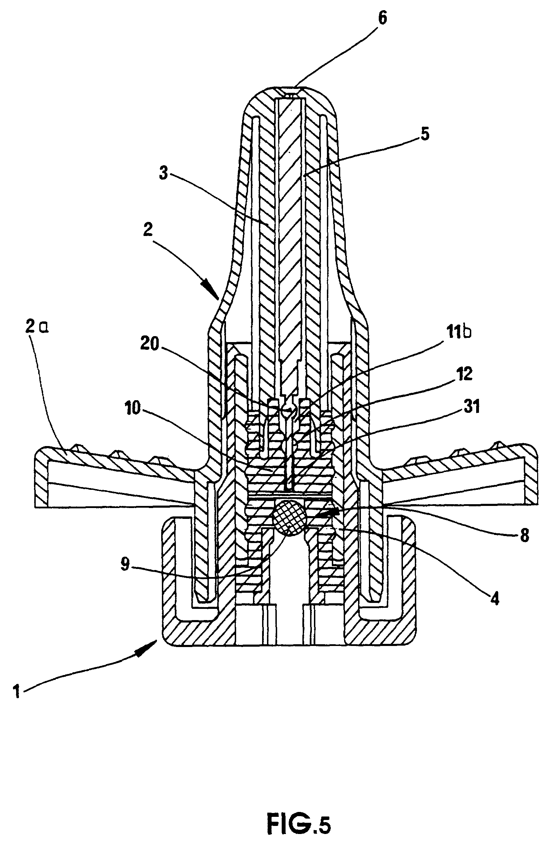

Le dispositif de distribution selon l'invention est représenté sur les figures 4 et 5. L'exemple de réalisation des figures 1 à 3 n'est pas couvert par l'invention, mais est décrit à titre explicatif.

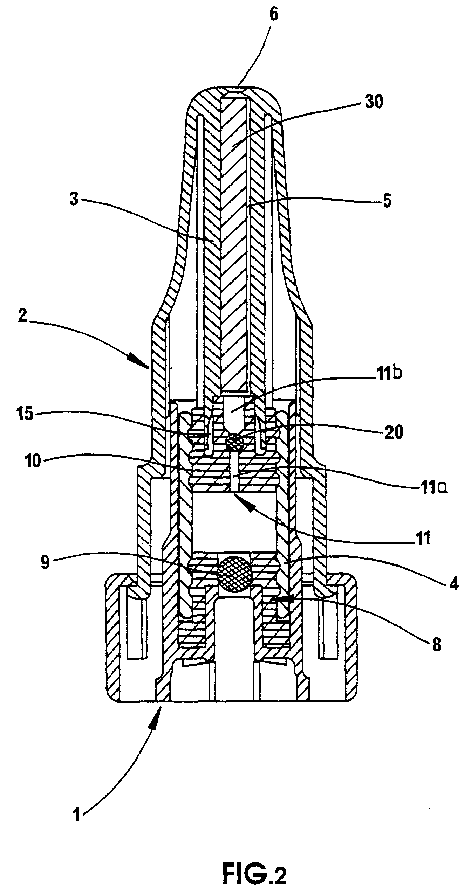

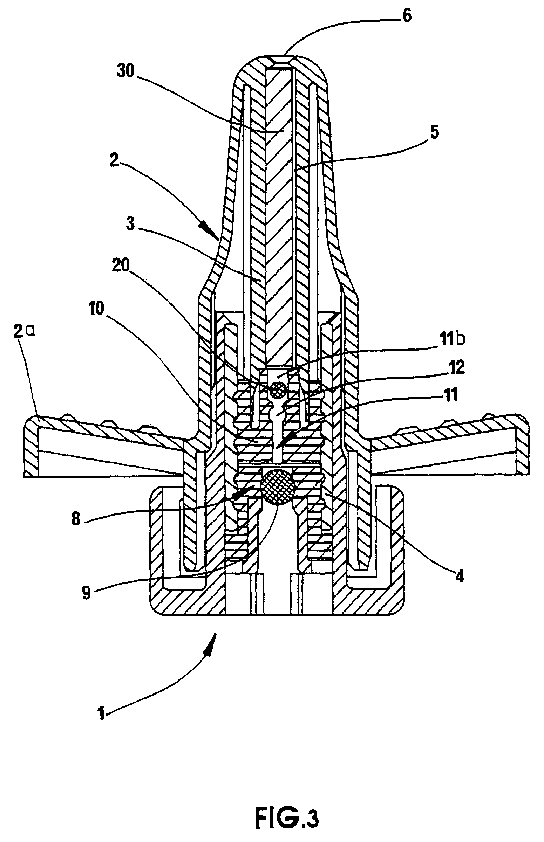

Le dispositif de distribution comporte avantageusement une forme extérieure similaire au dispositif décrit dans le document WC 93/00172. Ainsi, le dispositif comporte deux parties principales, mobiles l'une par rapport à l'autre une première partie 1 pouvant coulisser dans une seconde partie 2. La première partie 1, qui est la partie inférieure lorsque le dispositif est dans la position représentée sur les figures, comporte un réservoir 4, qui peut être avantageusement réalisé sous la forme d'un tube cylindrique fixé à ladite partie inférieure 1. Eventuellement le réservoir 4 peut faire parti intégrante de ladite partie inférieure 1. Toutefois il est avantageux pour certains produits de réaliser ce réservoir 4 en un matériau inerte vis à vis du produit, par exemple en verre. Ce réservoir 4 est obturé d'un côté de manière totalement étanche par un bouchon 8. Avantageusement, ce bouchon 8 est réalisé en un matériau élastomère assurant une bonne étanchéité et comporte un canal central permettant de remplir le réservoir avec le produit, ledit canal étant, après remplissage, obturé par un élément approprié tel qu'une bille 9. Toutefois, il est aussi possible de prévoir un bouchon 8 ne comportant pas de canal de remplissage, auquel cas, le produit peut être rempli dans le réservoir par l'intermédiaire d'une aiguille de remplissage qui viendrait percer le matériau élastomère du bouchon 8 pour remplir le réservoir 4 avec le produit, une aiguille de dégazage étant simultanément prévue pour évacuer l'air contenu initialement dans ce réservoir 4. Le côté du réservoir 4 obturé par le bouchon 8 est, sur les figures, le côté inférieur. La partie inférieure 1 du dispositif est adaptée à coulisser à l'intérieur de la partie supérieure 2, lors de l'actionnement du dispositif. La partie supérieure 2 comporte un organe d'actionnement 3, tel qu'une tige solidaire de la partie supérieure 2, et qui agit sur un piston 10, monté coulissant à l'intérieur du réservoir 4, du côté opposé au bouchon 8. Le piston 10 est adapté à se déplacer dans le réservoir 4 entre une position de stockage, représentée sur les figure 1, 2 et 4, dans laquelle il isole complètement le réservoir 4 vis à vis de l'extérieur, et une position d'actionnement, représentée sur les figures 3 et 5, dans laquelle le produit contenu dans le réservoir 4 a été distribuer par le dispositif de distribution. Pour actionner le dispositif, la partie supérieure 2 comporte avantageusement des ailettes d'actionnement 2a s'étendant latéralement de par et d'autre de la partie supérieure 2 et sur lesquelles l'utilisateur appuie avec ses doigts pour actionner le dispositif avec ses doigts, tout en maintenant son pouce sur la partie inférieure 1 de sorte que le dispositif peut être actionné manuellement de manière très simple. Comme représenté sur les figures 1 et 2, ces ailettes d'actionnement 2a peuvent ne s'étendre que dans un plan transversal du dispositif, représenté sur la figure 1 et non pas sur toute la périphérie de ladite partie supérieure 2. Cette partie supérieure 2 comporte en outre un passage de sortie 5 destiné à relier le réservoir 4 à un orifice de sortie 6. D'autre part, un gicleur 30 est avantageusement disposé à proximité dudit orifice 6 pour garantir une bonne pulvérisation de la dose de produit contenu dans le réservoir 4.

Dans la position de stockage, représentée sur les figures 1, 2 et 4, le réservoir contient donc une dose de produit et est obturé d'une part par le bouchon étanche 8 et d'autre part par ledit piston 10, également de manière étanche. Ainsi le produit contenu dans le réservoir 4 ne peut pas être oxydé ou pollué par l'air extérieur pendant toute la période du stockage. Dans la position de stockage, le piston 10 coupe donc la communication entre le réservoir 4 et le passage de sortie 5.

Le piston 10 comporte un passage traversant 11 qui relie le réservoir 4 au passage de sortie 5. Ce passage traversant 11 est obturé de manière étanche dans la position de stockage du piston 10 par un organe d'obturation 20. Cet organe d'obturation est adapté à ouvrir ledit passage traversant 11 sous l'effet d'une pression d'un niveau prédéterminé créé à l'intérieur du réservoir 4 lors de l'actionnement de du dispositif, de telle manière à permettre l'expulsion de ladite dose de produit fluide à travers ledit passage traversant 11, puis à travers ledit passage de sortie 5 en direction de l'orifice de sortie 6. De préférence, ledit passage traversant 11 s'étend axialement sensiblement au milieu dudit piston 10 et a une forme au moins partiellement environ cylindrique. L'organe d'obturation 20 est avantageusement mobile entre une position d'obturation, dans laquelle il est disposé à l'intérieur d'une première partie 11a dudit passage traversant 11, et une position d'ouverture, dans laquelle il est expulsé hors de ladite première partie 11a dudit passage traversant 11, sous l'effet de ladite pression d'un niveau prédéterminé régnant à l'intérieur du réservoir 4.

Avantageusement, le passage traversant 11 comporte deux parties 11a et 11b. La première partie 11a reçoit l'organe d'obturation dans sa position d'obturation et la seconde partie 11b le reçoit dans sa position d'ouverture. Dans ce cas, la section transversale de la seconde partie 11b est supérieure aux dimensions extérieures de l'organe d'obturation 20, pour permettre un écoulement du fluide à travers le passage traversant 11 lorsque l'organe d'obturation 20 est dans sa position d'ouverture. De même, l'organe d'obturation 20 a des dimensions extérieures légèrement supérieures à la section transversale de la première partie 11a du passage traversant 11, de sorte que ledit organe d'obturation 20 peut être maintenu dans sa position d'obturation par coincement. Avantageusement, comme visible sur les figures 3 et 5, le passage traversant 11 peut comporter une cavité 12, destinée à recevoir ledit organe d'obturation 20. Cette cavité 12 a avantageusement une forme identique à celle de l'organe d'obturation 20 et est adapté à le maintenir dans sa position d'obturation de manière étanche. Cette mise en oeuvre facilite la mise en place de l'organe d'obturation 20 dans le passage traversant 11 lors du montage du dispositif, et permet de déterminer avec plus de précision le niveau prédéterminé de pression nécessaire à l'expulsion dudit organe d'obturation 20.

Après actionnement du dispositif comme représenté sur les figures 3 et 5, l'organe d'obturation 20 se retrouve dans ladite seconde partie 11b du passage traversant 11 et ce passage traversant 11 n'est plus obturé et relie l'intérieur du réservoir 4 au passage de sortie 5 pour permettre l'expulsion du produit. Eventuellement, l'organe d'obturation 20 pourrait, dans sa position d'ouverture, se retrouver complètement hors du passage traversant 11 du piston 10. Dans ce cas le passage traversant 11 n'est pas nécessairement réalisé avec deux parties 11a et 11b de sections transversales différentes.

Lorsque l'utilisateur actionne le dispositif, il exerce une pression pour déplacer la partie inférieure 1 du dispositif par rapport à la partie supérieure 2 du dispositif. Le produit fluide contenu dans le réservoir 4 étant incompressible, cette pression est donc transmise directement par ledit produit à l'organe d'obturation 20 disposé dans le canal traversant 11. Lorsque la pression atteint un niveau prédéterminé suffisant, l'organe d'obturation 20 est expulsé hors de ladite première partie 11a du passage traversant 11 et le produit contenu dans le réservoir est expulsé à travers ledit passage traversant en direction du passage de sortie 5 puis de l'orifice de sortie 6 du dispositif. Avantageusement, les parois du piston 10 qui forment le canal traversant 11 sont déformables pour permettre l'expulsion dudit organe d'obturation 20 sous l'effet de la pression. Ainsi, en référence aux figures, on peut prévoir un espace radial 15 disposé autour desdites parois formant le canal traversant 11 du piston 10 de sorte que ces parois du piston 10 peuvent se déformer radialement en direction de cet espace 15 pour permettre l'expulsion de l'organe d'obturation 20 hors du passage traversant 11.

Le piston 10 est avantageusement fixe par rapport à l'organe d'actionnement 3 et se déplace ensemble avec ledit organe d'actionnement 3 dans le réservoir 4 lors de l'actionnement du dispositif. La fabrication et le montage du distributeur selon l'invention est donc grandement simplifié ce qui entraîne une baisse des coûts non négligeable.
En référence aux figures 1 à 3, il est représenté un premier mode de réalisation qui n'est pas couvert par l'invention, où l'organe d'obturation 20 est réalisé sous la forme d'une bille qui est insérée dans le canal traversant 11.

En référence aux figures 4 et 5, il est représenté un mode de réalisation préféré de l'invention, où l'organe d'obturation 20 est solidaire du gicleur 30. Dans ce cas, le gicleur 30 est mobile entre une position initiale, représentée sur la figure 4, correspondant à la position d'obturation de l'organe d'obturation 20, et une position finale, représentée sur la figure 5 correspondant à la position d'ouverture de l'organe d'obturation 20. Cette mise en oeuvre permet d'économiser une pièce constitutive du dispositif et facilite d'avantage encore la fabrication et le montage de ce dispositif, d'où économie des coûts de fabrication et de montage. Comme représenté sur la figure 4, ledit organe d'obturation 20 peut avantageusement être formé par une partie, par exemple sphérique, du gicleur 30, dont les dimensions extérieures sont supérieures aux dimensions intérieurs de la première partie 11a du passage traversant 11. Cette partie sphérique 20 est reliée au gicleur 30 par une partie de tige de dimension appropriée, c'est à dire qui remplit au maximum les volumes morts dans la position initiale de la figure 4 et qui permet un passage du produit lors de l'actionnement du dispositif. Selon une variante avantageuse, le gicleur 30 peut comporter en outre une partie d'extrémité 31 qui s'étend dans le réservoir 4 lorsque le gicleur 30 est dans sa positon initiale, telle que représentée sur la figure 4. Cette partie d'extrémité 31 est destiné à remplir au moins partiellement le volume mort créé dans le passage traversant 11 lorsque le gicleur 30 est dans sa position finale représentée sur la figure 5. Ceci favorise encore d'avantage une expulsion totale et complète de la dose de produit contenu dans le réservoir 4.

Ainsi, lorsque l'utilisateur actionne le dispositif, il créé une pression à l'intérieur du réservoir qui, puisque le produit fluide est incompressible, est transmise à la partie sphérique 20 du gicleur 30 formant l'organe d'obturation, et qui est maintenu avantageusement dans ladite cavité 12 dans ledit passage traversant 11. Lorsque ladite pression atteint un niveau prédéterminé suffisant, ladite partie sphérique 20 est expulsée hors de la cavité 12 et l'ensemble du gicleur 30 se déplace alors de sa position initiale vers sa position finale, dans laquelle il garantit une bonne pulvérisation de la dose de produit.

L'invention permet donc de réaliser un dispositif de distribution d'une dose unique de produit fluide, communément appelé mono-spray, d'une manière simple et peu coûteuse. En particulier, le dispositif de l'invention garantit une expulsion totale et complète de la dose de produit contenu dans le réservoir 4 en raison de la pré-compression qu'il engendre. Ainsi l'utilisateur, lorsque qu'il actionne le dispositif, emmagasine une certaine quantité d'énergie dans la main jusqu'à ce qu'il créé ladite pression suffisante à expulser l'organe d'obturation hors du.passage traversant du piston. Cette énergie emmagasinée dans la main de l'utilisateur a pour effet, que lorsque l'organe d'obturation est expulsé hors du passage traversant, le piston coulisse dans le réservoir de manière brusque et rapide de sa positon de stockage vers sa position d'actionnement de sorte que le produit contenu dans le réservoir est complètement expulsé.

## Revendications

1. Dispositif de distribution d'une dose unique de produit fluide, comportant :
- un réservoir (4) contenant ladite dose de produit fluide,
- un piston (10) coulissant de manière étanche dans ledit réservoir (4) entre une position de stockage dans laquelle il isole ledit réservoir, et une position d'actionnement,
- un organe d'actionnement (3) du piston,
- un passage de sortie (5) pour relier ledit réservoir (4) à un orifice de sortie (6), et
- un gicleur (30) disposé dans le passage de sortie (5) pour pulvériser la dose de produit,
ledit piston (10) comportant un passage traversant (11) qui est obturé de manière étanche, dans la position de stockage du piston (10), par un organe d'obturation (20), ledit organe d'obturation étant adapté à ouvrir ledit passage traversant (11) sous l'effet d'une pression d'un niveau prédéterminé créée à l'intérieur du réservoir (4), de telle manière à permettre l'expulsion de ladite dose de produit fluide à travers ledit passage traversant (11), **caractérisé en ce que** ledit organe d'obturation (20) est solidaire dudit gicleur (30), ledit gicleur (30) étant mobile entre une position initiale correspondant à la position d'obturation de l'organe d'obturation (20), et une position finale correspondant à la position d'ouverture de l'organe d'obturation (20).

2. Dispositif selon la revendication 1, dans lequel ledit organe d'obturation (20) est mobile entre une position d'obturation, dans laquelle il est disposé dans une première partie (11a) dudit passage traversant (11), et une position d'ouverture, dans laquelle il est expulsé hors de ladite première partie (11a) du passage traversant (11).

3. Dispositif selon la revendication 2, dans lequel ledit passage traversant (11) est au moins partiellement cylindrique et ledit organe d'obturation (20) a des dimensions extérieures supérieures à la section transversale de ladite première partie (11a) du passage traversant (11), de sorte qu'il est maintenu en position d'obturation par coincement, ledit organe d'obturation (20) étant expulsé hors de ladite première partie (11a) du passage traversant (11) sous l'effet de ladite pression suffisante créée dans le réservoir (4).

4. Dispositif selon la revendication 2 ou 3, dans lequel ledit passage traversant (11) comporte une cavité (12) adapté à recevoir ledit organe d'obturation (20) dans sa position d'obturation.

5. Dispositif selon l'une quelconque des revendications 2 à 4, dans lequel les parois formant le passage traversant (11) du piston (10) sont déformables sous l'effet d'une pression suffisante pour permettre l'expulsion dudit organe d'obturation (20).

6. Dispositif selon l'une quelconque des revendications précédentes, dans lequel le gicleur (30) comporte une partie d'extrémité (31) s'étendant dans ledit réservoir (4) lorsque le gicleur (30) est dans sa position initiale, ladite partie d'extrémité (31) étant destinée à remplir au moins partiellement le volume mort créé dans le passage traversant (11) lorsque le gicleur (30) est dans sa position finale.

7. Dispositif selon l'une quelconque des revendications précédentes, dans lequel ledit passage traversant (11) comporte une première partie (11a) sensiblement cylindrique et une seconde partie (11b) sensiblement cylindrique, la section transversale de ladite seconde partie (11b) étant supérieure à celle de ladite première partie (11a), ledit organe d'obturation (20) ayant des dimensions extérieures qui sont supérieures à ladite section transversale de la première partie (11a) et inférieures à ladite section transversale de la seconde partie (11b).

8. Dispositif selon l'une quelconque des revendications précédentes, dans lequel ledit réservoir (4) est en verre.

9. Dispositif selon l'une quelconque des revendications précédentes, dans lequel ledit piston (10) est fixe par rapport audit organe d'actionnement (3) et se déplace dans le réservoir (4) ensemble avec ledit organe d'actionnement (3).

## Patentansprüche

1. Abgabevorrichtung für eine einzige Dosis eines Fluids, die folgende Bestandteile umfaßt:
- einen Behälter (4) der die Dosis des Fluids enthält
- einen Kolben (10) der in dichter Weise im Behälter (4) zwischen einer Speicherposition, in welcher er den Behälter dicht verschließt, und einer Betätigungsposition gleitet,
- ein Betätigungsorgan (3) für den Kolben,
- einen Ausgangs-Durchgang (5), der den Behälter (4) mit einer Ausgangsöffnung (6) verbindet, und
- eine Spritzdüse (30), die im Ausgangs-Durchgang (5) angeordnet ist, um die Produkt-Dosis zu zerstäuben,
wobei der Kolben (10) einen Durchgangskanal (11) umfaßt, der in der SpeicherPosition (10) des Kolbens in dichter Weise durch ein Verschlußorgan (20) verschlossen ist, wobei das Verschlußorgan geeignet ist, den Durchgangskanal (11) unter der Wirkung eines Drucks mit vorbestimmter Größe zu öffnen, der im Inneren des Behälters (4) erzeugt wird, derart, daß das Ausstoßen der Dosis des Fluids durch den Durchgangskanal (11) ermöglicht wird,
**dadurch gekennzeichnet, daß** das Verschlußorgan (20) mit der Spritzdüse (30) fest verbunden ist, wobei die Spritzdüse (30) zwischen einer Anfangsposition, die der Verschlußposition des Verschlußorgans (20) entspricht, und einer Endposition bewegbar ist, die der Öffnungsposition des Verschlußorgans (20) entspricht.

2. Vorrichtung nach Anspruch 1, bei der das Verschlußorgan (20) zwischen einer Verschlußposition, in welcher es in einem ersten Teil (11a) des Durchgangskanals (11) angeordnet ist, und einer Öffnungsposition bewegbar ist, in welcher es aus dem ersten Teil (11a) des Durchgangskanals (11) ausgestoßen ist.

3. Vorrichtung nach Anspruch 2, bei der der Durchgangskanal (11) zumindest teilweise zylindrisch ausgebildet ist und das Verschlußorgan (20) Außenabmessungen besitzt, die größer sind als der quer verlaufende Schnitt des ersten Teils (11a) des Durchgangskanals (11) derart, daß es durch Einklemmen in seiner Verschlußposition gehalten wird, wobei das Verschlußorgan (20) aus dem ersten Teil (11a) des Durchgangskanals (11) unter der Wirkung des in ausreichendem Maße im Behälter (4) erzeugten Drucks ausgestoßen wird.

4. Vorrichtung nach Anspruch 2 oder 3, bei der der Durchgangskanal (11) einen Hohlraum (12) umfaßt, der geeignet ist, das Verschlußorgan (20) in seiner Verschlußstellung aufzunehmen.

5. Vorrichtung nach einem der Ansprüche 2 bis 4, bei dem die Wände, die den Durchgangskanal (11) des Kolbens (10) bilden, unter der Wirkung eines ausreichenden Drucks verformbar sind, um das Ausstoßen des Verschlußorgans (20) zu ermöglichen.

6. Vorrichtung nach einem der vorhergehenden Ansprüche, bei der die Spritzdüse (30) einen Endteil (31) umfaßt, der sich in den Behälter (4) hinein erstreckt, wenn sich die Spritzdüse (30) in ihrer Anfangsposition befindet, wobei der Endteil (31) dazu bestimmt ist, zumindest teilweise das Totvolumen auszufüllen, das im Durchgangskanal (11) erzeugt wird, wenn sich die Spritzdüse (30) in ihrer Endposition befindet.

7. Vorrichtung nach einem der vorhergehenden Ansprüche, bei der der Durchgangskanal (11) einen im wesentlichen zylindrischen ersten Teil (11a) und einen im wesentlichen zylindrischen zweiten Teil (11b) umfaßt, wobei der quer verlaufende Schnitt des zweiten Teils (11b) größer ist als der des ersten Teils (11a), und wobei das Verschlußorgan (20) Außenabmessungen besitzt, die größer sind, als der quer verlaufende Schnitt des ersten Teils (11a) und kleiner als die des quer verlaufenden Schnitts des zweiten Teils (11b).

8. Vorrichtung nach einem der vorhergehenden Ansprüche, bei der der Behälter (4) aus Glas besteht.

9. Vorrichtung nach einem der vorhergehenden Ansprüche, bei der der Kolben (10) bezüglich des Betätigungsorgans (3) fest angeordnet ist und sich in den Behälter (4) zusammen mit dem Betätigungsorgan (3) verschiebt.

## Claims

1. A dispenser device for dispensing a single dose of liquid, said device comprising:
a cylinder (4) containing said dose of liquid;
a piston (10) which slides in sealed manner in said cylinder (4) between a storage position in which it isolates said cylinder and an actuated position;
a piston actuator member (3) ;
an outlet passage (5) for connecting said cylinder (4) to an outlet orifice (6); and
a spray nozzle (30) which is disposed in the outlet passage (5) for spraying the dose of liquid;
said piston (10) including a through-passage (11) which is closed in sealed manner in the storage position of the piston (10) by a closure member (20), said closure member being designed to open said through-passage (11) under the effect of a predetermined pressure which is created inside the cylinder (4), so as to enable said dose of liquid to be expelled through said through-passage (11), the device being **characterized in that** said closure member (20) is secured to said spray nozzle (30), said spray nozzle (30) being movable between an initial position corresponding to the closed position of the closure member (20), and a final position corresponding to the open position of the closure member (20).

2. A device according to claim 1, in which said closure member (20) is movable between a closed position, in which it is disposed in a first portion (11a) of said through-passage (11), and an open position, in which it is expelled out from said first portion (11a) of the through-passage (11).

3. A device according to claim 2, in which said through-passage (11) is at least partially cylindrical, and said closure member (20) has outside dimensions that are greater than the cross section of said first portion (11a) of the through-passage (11) so that it is blocked in the closed position, said closure member (20) being expelled out from said first portion (11a) of the through-passage (11) under the effect of said sufficient pressure which is created in the cylinder (4).

4. A device according to claim 2 or 3, in which said through-passage (11) includes a cavity (12) designed to receive said closure member (20) in its closed position.

5. A device according to any one of claims 2 to 4, in which the walls which form the through-passage (11) of the piston (10) are deformable under the effect of a pressure that is sufficient to enable said closure member (20) to be expelled.

6. A device according to any preceding claim, in which the spray nozzle (30) includes an end portion (31) which extends into said cylinder (4) when the spray nozzle (30) is in its initial position, said end portion (31) being designed to occupy, at least in part, the empty space created in the through-passage (11) when the spray nozzle (30) is in its final position.

7. A device according to any preceding claim, in which said through-passage (11) includes a substantially cylindrical first portion (11a) and a substantially cylindrical second portion (11b), the cross section of said second portion (11b) being greater than that of said first portion (11a), said closure member (20) having outside dimensions which are greater than said cross section of the first portion (11a) and which are less than said cross section of the second portion (11b).

8. A device according to any preceding claim, in which said cylinder (4) is made of glass.

9. A device according to any preceding claim, in which said piston (10) is fixed relative to said actuator member (3) and is displaced in the cylinder (4) together with said actuator member (3).
